# FASCICULE DE BREVET EUROPEEN

(11) **EP 3 784 082 B1**
(45) Date de publication et mention de la délivrance du brevet: **17.01.2024**
(21) Numéro de dépôt: 19730211.0
(22) Date de dépôt: 23.04.2019
(51) Int. Cl.: A41D 13/018, A41D 13/05, A61B 5/01, A61B 5/11

(54) **DISPOSITIF DE SECURITE A AIRBAG**
AIRBAG-SICHERHEITSVORRICHTUNG
AIRBAG SAFETY DEVICE

(30) Priorité: 26.04.2018 CH 5362018; 11.05.2018 CH 5882018
(43) Date de publication de la demande: 03.03.2021
(73) Titulaire: Service a la Personne Technologie Active Sarl, 1219 Chatelaine (CH)
(72) Inventeur: JEANDUPEUX, Thierry, 74520 Dingy en Vuache (FR)
(74) Mandataire: Cronin, Brian Harold John
(86) Numéro de dépôt international: PCT/IB2019/053333
(87) Numéro de publication internationale: WO 2019/207474

(56) Documents cités:
- WO-A1-2017/151645
- CN-U- 205 041 401
- US-A1- 2005 067 816
- US-A1- 2017 208 874
- US-A1- 2017 277 158
- US-B2- 6 828 697

## Description

La présente invention se rapporte à un dispositif de sécurité à airbag pour prévenir des blessures aux hanches, au bassin, aux fesses et au coccyx en cas de chute.

US2015351666A1 décrit un système d'analyse du mouvement comportant au moins un capteur d'orientation configuré pour détecter le mouvement du torse en trois dimensions dans le temps, de même qu'un airbag se déclenchant lors d'une chute afin d'amortir le corps de l'utilisateur. Ce dispositif est contraignant et inconfortable dans la mesure où une pluralité de capteurs est répartie sur le système dont un capteur disposé sur le cou de l'utilisateur.

US6920647B2 décrit un dispositif de protection de la hanche permettant de minimiser le risque de rupture de la hanche lors d'une chute. Ce dispositif possède un système permettant de détecter le taux et le degré de changement d'attitude de l'utilisateur permettant ainsi au dispositif de s'enclencher lors d'une éventuelle chute afin de gonfler des poches et amortir la chute.

WO2017/151645 décrit dispositif de protection comprenant un ensemble de coussin gonflable configuré pour s'étendre au moins partiellement autour de la taille ou des hanches d'un individu, une boucle fixée à l'ensemble du coussin gonflable, la boucle comprenant une première moitié de boucle, et une deuxième moitié de boucle, les première et deuxième moitiés de boucle pouvant être attachées et détachées l'une de l'autre. Le dispositif comprend un actionneur de coussin gonflable configuré pour actionner l'ensemble du coussin gonflable. Une partie de l'actionneur de coussin gonflable est disposée dans la première moitié de boucle ou la deuxième moitié de la boucle. Un inconvénient de cette réalisation est que le dispositif peut se déclencher de manière inopinée même s'il n'est pas porté par un utilisateur autour de la ceinture mais par exemple juste porté à la main pour être rangé.

Le document US2017208874 décrit un dispositif de protection avec les caractéristiques du préambule de la revendication 1.

Le but de la présente invention est de proposer un dispositif de sécurité à airbag peu contraignant pour l'utilisateur et qui ne se déclenche pas de manière inopinée.

L'invention concerne un dispositif de sécurité du type pour prévenir les blessures aux hanches, au bassin, aux fesses et au coccyx par gonflage d'airbag adapté à un porteur comprend : une poche gonflable portative, couplable à un vêtement ; au moins une source de gaz libérable couplée à la poche ; une boucle de ceinture dans laquelle sont agencés une pluralité de capteurs permettant de déterminer le mouvement angulaire et l'accélération d'un porteur lors d'une chute; un microcontrôleur adapté pour analyser les données reçues par les différents blocs fonctionnels pour définir le moment où déclencher la source de gaz ; et un moyen pour libérer le gaz de la source dans la poche selon une détermination du microcontrôleur que le porteur tombe.

Selon l'invention, deux des capteurs agencés dans la boucle sont un premier capteur thermique mesurant la température ambiante et un second capteur thermique mesurant la température corporelle d'un porteur. Les premiers et seconds capteurs thermiques sont agencés pour autoriser un déclenchement de l'airbag en cas de chute seulement lorsque le dispositif est porté par un porteur, notamment par la mesure de la différence de température mesurée par les deux capteurs thermiques s'assurant ainsi de la présence d'un porteur.

Dans une forme d'exécution, la boucle comporte au moins une paire d'aimants, de préférence deux paires, pour le verrouillage de la boucle.

Dans une autre forme d'exécution, la boucle comporte un contacteur à piston pour l'activation du dispositif, une fois la ceinture fermée.

Dans une forme d'exécution, le dispositif comporte une source d'énergie rechargeable à travers la boucle, notamment par cordon USB.

Dans toutes les formes d'exécution, le dispositif peut comporter un module inertiel six axes permettant de détecter les mouvements d'un utilisateur le module inertiel comportant notamment un accéléromètre couplé à un gyroscope.

Selon une forme d'exécution particulière la boucle comporte une ouverture agencée pour laisser passer un fil électrique disposé pour transmettre une impulsion électrique donnée par le microcontrôleur pour autoriser un déclenchement de l'airbag en cas de chute.

Dans une forme d'exécution, des moyens visuels et/ou sonores sont agencés pour indiquer l'état de fonctionnement dudit dispositif.

De préférence, la boucle comprend (A) une première partie de forme cylindrique comportant une rainure hélicoïdale s'étendant d'une extrémité de la première partie de boucle, la rainure étant légèrement courbée avec une encoche à sa seconde extrémité, et (B) une seconde partie comportant une protubérance agencée pour coulisser dans ladite rainure et venir s'engager dans ladite encoche. Chaque partie de la boucle comporte un aimant agencé pour solidariser la première et la seconde partie de boucle, les deux dites parties de boucle étant désolidarisables l'une de l'autre par le déplacement angulaire, notamment d'au moins un huitième de tour d'une première partie de boucle par rapport à l'autre partie de boucle puis par le coulissement de la protubérance dans la rainure jusqu'à son extraction totale.

Une telle boucle, pourvue ou non d'électronique peut aisément être agencée sur d'autres formes de ceintures pour notamment faciliter l'ouverture et la fermeture de la boucle.

Dans une forme d'exécution, la boucle est notamment formée de deux parties assemblables, une première partie en forme de boîtier rectangulaire contenant l'électronique et une deuxième partie aussi rectangulaire, ces deux parties comportant ensemble un corps cylindrique de connexion comportant un dispositif de fixation à baïonnette. Les côtés externes desdites parties de cette boucle comportent des ouvertures pour s'attacher aux extrémités de la poche formant ceinture.

Pour renforcer la sécurité du porteur, le dispositif comporte un module de communication sans-fil, notamment par liaison radio, apte à transmettre une information relative au déclenchement de l'airbag à une autre personne que le porteur dudit dispositif. Le module de communication est apte à transmettre une information relative à un état du dispositif correspondant à la présence ou non d'un porteur et au déclenchement ou non de l'airbag.

Selon un mode de réalisation de l'invention, le module de communication permet également de géolocaliser le porteur. Le dispositif peut notamment fournir, grâce à un système de supervision correspondant, d'une part un état du dispositif qu'une tierce personne, par exemple un médecin ou un parent, peut contrôler à tout moment et en temps réel via la communication et d'autre part la géolocalisation du porteur.

De préférence, le module de communication est un module basse consommation de façon à minimiser sa consommation électrique.

Dans une forme d'exécution, le microcontrôleur stocke un algorithme qui enregistre chaque mouvement que l'on peut appeler de non standard c'est-à-dire chaque mouvement qui pourrait correspondre soit à une chute soit à un mouvement très brusque mais qui n'est pas une chute. Ainsi, en déterminant de manière précise un mouvement correspondant à une chute, le microcontrôleur pourra définir de manière précise et pertinente le moment où déclencher la source de gaz et libérer le gaz de la source dans la poche. A intervalle régulier, le microcontrôleur recharge donc un nouvel algorithme amélioré et optimisé. De préférence, cette mise à jour du logiciel est réalisée lorsque l'utilisateur connecte son dispositif de sécurité à un ordinateur. Dans une variante, la mise à jour du logiciel peut être faite à distance par un technicien qui se connecterait au dispositif de sécurité.

De préférence, à chaque déclenchement de l'airbag, le dispositif est configuré pour envoyer un signal d'alerte, notamment un SMS ou un appel téléphonique d'urgence pour alerter une tierce personne. Le dispositif de sécurité peut être connecté via une connexion internet sans fil à une application apte à relayer les alertes par SMS et/ou e-mail associés à des services compétents. Le dispositif de sécurité peut également être connecté au téléphone cellulaire d'un utilisateur qui peut relayer les alertes d'une part et la position de l'utilisateur d'autre part en utilisant une connexion cellulaire et les coordonnées GPS.

Dans une forme d'exécution, le dispositif de sécurité comporte sur sa boucle une commande tel un bouton poussoir configuré pour envoyer une alerte à une tierce personne tel un soignant.

Pour renforcer la sécurité, la source de gaz peut être agencée dans une poche qui la retient de manière à ne pas être propulsée en cas de déclenchement de l'airbag (sécurisation supplémentaire).

Les caractéristiques de l'invention apparaitront plus clairement à la lecture de la description d'une forme d'exécution donnée uniquement à titre d'exemple, nullement limitative en se référant aux figures schématiques, dans lesquelles :
La figure 1 est un diagramme schématique montrant un dispositif de sécurité à airbag porté par un porteur ;
La figure 2 est une vue en perspective de dessus d'une boucle du dispositif de sécurité à airbag ;
La figure 3 est une vue en perspective de dessous de la boucle du dispositif de sécurité à airbag ;
La figure 4 est une vue de côté de la boucle du dispositif de sécurité à airbag ;
La figure 5 est une vue en perspective d'une partie de la boucle du dispositif de sécurité à airbag ;
La figure 6 est une vue en perspective d'une autre partie du dispositif de sécurité à airbag ; et
Les figures 7 et 8 sont des vues en perspective d'une autre version de boucle du dispositif de sécurité à airbag.

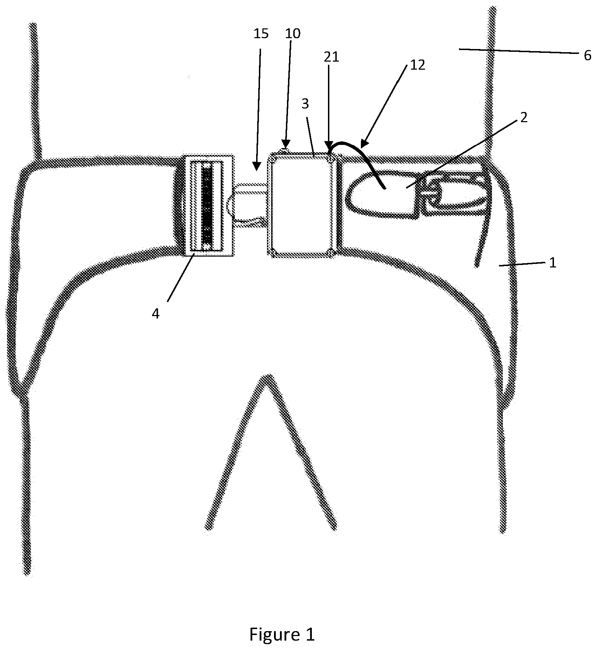

La figure 1 montre un dispositif de sécurité à airbag selon l'invention porté par un porteur 6 de manière à prévenir les blessures aux hanches, au bassin, aux fesses et au coccyx par gonflage d'un airbag adapté aux hanches d'un porteur 6. Ce dispositif comprend une poche gonflable 1 portative couplable autour d'un vêtement du porteur 6.

La poche gonflable 1 porte une source 2 de gaz libérable couplée à la poche 1. La poche gonflable 1 est portée par une boucle de ceinture 3, 4 dans laquelle sont agencés une pluralité de capteurs permettant de déterminer le mouvement angulaire et l'accélération d'un porteur lors d'une chute. La boucle 3, 4 contient aussi un microcontrôleur adapté pour analyser les données reçues par les différents blocs fonctionnels pour définir le moment où déclencher la source 2 de gaz ainsi qu'un moyen pour libérer le gaz de la source 2 pour remplir la poche 1 selon une détermination du microcontrôleur que le porteur 6 tombe. Le microcontrôleur constitue le coeur de l'appareil exécutant l'algorithme de détection et gère tous les autres blocs. Une fois toutes les conditions requises (ceinture fermée, personne détectée, sens d'insertion correct), c'est ce composant qui analysera les données du module inertiel afin de définir le moment où déclencher l'airbag.

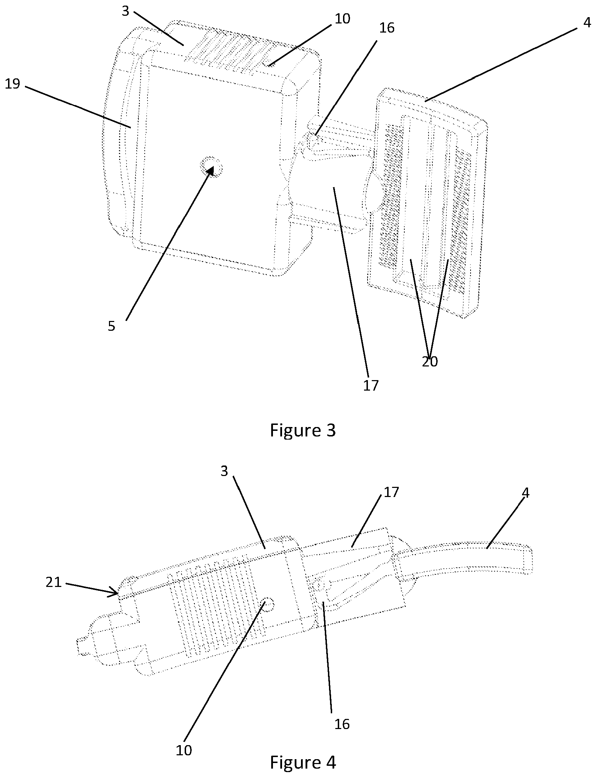

Un des capteurs agencés dans la partie 3 de la boucle est un capteur thermique 5 (figure 3) agencé du côté intérieur de cette partie 3 afin de mesurer la température corporelle du porteur 6 couplé avec un capteur de température ambiant agencé à l'intérieur du boitier de la boucle, et donc non visible au dessin, pour fournir un signal pour autoriser un déclenchement de l'airbag en cas de chute seulement lorsque le dispositif est porté par un porteur 6. Le capteur thermique 5 est un thermomètre infrarouge permettant de mesurer la température d'un corps distant notamment de détecter la chaleur humaine du porteur 6. Couplé avec la mesure de la température ambiante, le système indique si la ceinture est bien portée par une personne plutôt que simplement rangée en position fermée.

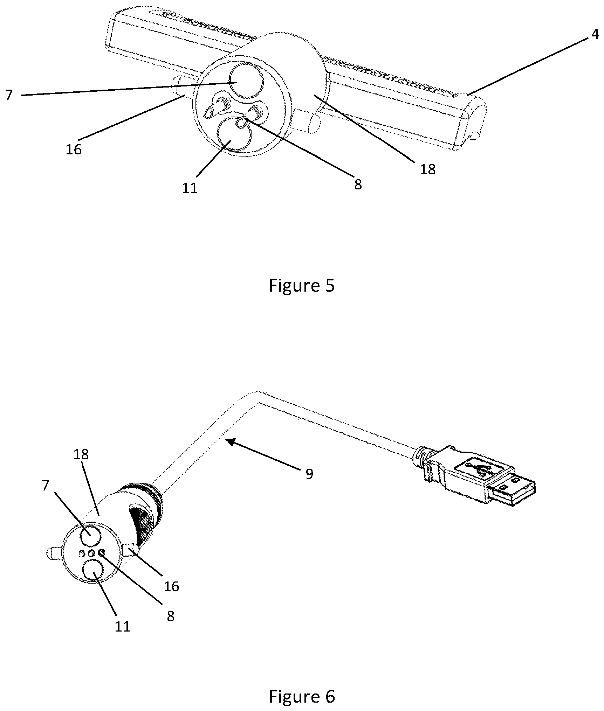

Comme illustrée à la figure 5, l'extrémité interne de la partie 4 de la boucle comporte deux aimants 7,11 pour le verrouillage de la boucle et un contact à pistons 8 à ressorts pour l'activation du dispositif, une fois la ceinture fermée. L'électronique permet de gérer l'enclenchement de l'appareil. Des contacts à piston 8 à ressort sur chaque partie de boucle la boucle faisant contact sur chaque partie de boucle pour détecter que la boucle de la ceinture est fermée.

La figure 6 illustre une source d'énergie rechargeable à travers la partie de boucle 3, notamment par un cordon USB 9. Ceci permet de charger une batterie interne. Ce bloc peut être simplifié et un simple chargeur de batterie sera présent.

Le dispositif selon l'invention comporte typiquement un module inertiel six axes permettant de détecter les mouvements d'un utilisateur le module inertiel comportant notamment un accéléromètre couplé à un gyroscope. Ces éléments sont contenus dans la partie 3 de la boucle. Ce module permet de détecter de manière connue les mouvements de la personne et ainsi prédire la chute avant l'impact. L'accéléromètre couplé au gyroscope permet de différencier une vraie chute de simple vibration ou de mouvements normaux de la personne.

La partie 3 de la boucle comporte une ouverture 21 (figure 1) dans son côté, agencé pour laisser passer un fil électrique 12 disposé pour transmettre une impulsion électrique donnée par le microcontrôleur pour autoriser un déclenchement de l'airbag en cas de chute.

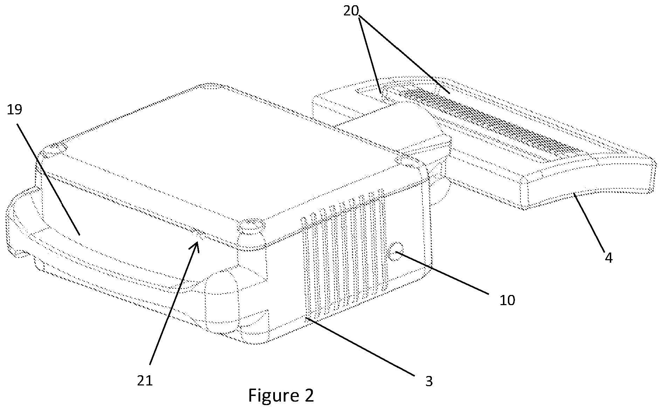

Dans cet exemple, une LED 10 (figure 2) et un buzzer non illustré permettent d'informer le porteur de l'état de fonctionnement du dispositif, notamment en émettant un clignotement de la LED 10 en cas de dysfonctionnement puis l'émission de plusieurs bips si le porteur n'a pas réagi aux signaux émis par la LED 10.

Le dispositif peut optionnellement comporter un dispositif auditif ou lumineux permettant d'indiquer à l'utilisateur d'autres situations que le dysfonctionnement. Par exemple : lorsque la ceinture activée est correctement portée : deux bips aigus, lorsque la ceinture est portée mais à l'envers : deux bips graves ; lorsque la ceinture est enlevée, extinction de l'appareil : bip grave, batterie faible : quatre bips graves rapides toutes les quinze minutes.

Comme illustrée, la boucle est formée de deux parties assemblables, une première partie 3 en forme de boîtier rectangulaire contenant l'électronique (notamment les capteurs ainsi que le microcontrôleur), et une deuxième partie 4 rectangulaire évidée. Ces deux parties 3, 4, comporte ensemble un corps cylindrique de connexion 15 comportant un dispositif de fixation à baïonnette 16, soit une partie cylindrique creuse 17 sur un côté de la partie 3 qui reçoit une partie cylindrique pleine 18 sur un côté de la partie 4. Les surfaces principales des parties 3,4 sont soit plates, ce qui est le cas pour la partie 3, soit légèrement courbées, ce qui est le cas pour la partie 4, pour pouvoir venir se plaquer contre le corps du porteur 6. Les côtés externes des parties 3 et 4 comportent des ouvertures allongées 19, 20 pour s'attacher aux extrémités de la poche 1 formant ceinture.

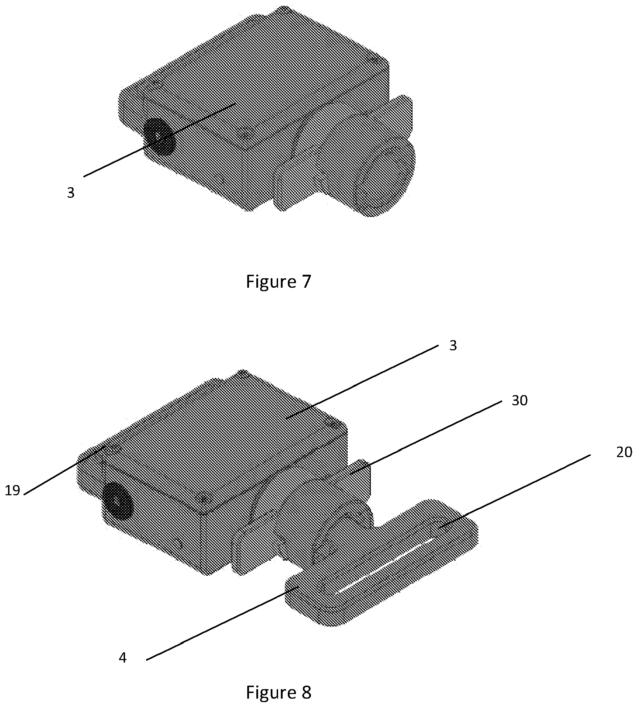

Comme illustré aux figures 7 et 8, la boucle est formée de deux parties assemblables, une première partie 3 en forme de boîtier rectangulaire contenant l'électronique, et une deuxième partie 4 rectangulaire évidée. Ces deux parties 3, 4, comporte ensemble un corps cylindrique de connexion comportant un dispositif de fixation comportant deux ailettes 30 facilitant l'ouverture de la boucle. Les côtés externes des parties 3 et 4 comportent des ouvertures allongées 19, 20 pour s'attacher aux extrémités de la poche formant ceinture.

## Revendications

1. Dispositif de sécurité pour prévenir les blessures aux hanches, au bassin, aux fesses et au coccyx par gonflage d'airbag adapté à un porteur (6), ledit dispositif comprenant :
- une poche gonflable (1) portative ;
- au moins une source (2) de gaz libérable couplée à la poche (1) ;
- une boucle de ceinture (3, 4) dans laquelle sont agencés une pluralité de capteurs permettant de déterminer le mouvement angulaire et l'accélération d'un porteur (6) lors d'une chute du porteur ;
- un microcontrôleur adapté pour analyser les données reçues par les différents blocs fonctionnels pour définir le moment où déclencher la source de gaz ; et
- un moyen pour décharger la source (2) de libérer le gaz dans la poche (1) selon une détermination du microcontrôleur que le porteur tombe ;
**caractérisé en ce que** :
deux des capteurs agencés dans la boucle sont un premier capteur thermique mesurant la température ambiante et un second capteur thermique (5) mesurant la température corporelle d'un porteur (6), les premiers et seconds capteurs thermiques étant agencés pour autoriser un déclenchement de l'airbag en cas de chute seulement lorsque le dispositif est porté par un porteur par la mesure de la différence de température mesurée par les deux capteurs thermiques s'assurant ainsi de la présence d'un porteur du dispositif et **en ce qu'**il comporte un module de communication sans-fil, apte à transmettre une information relative à un état du dispositif correspondant à la présence ou non d'un porteur et au déclenchement ou non de l'airbag à une autre personne que le porteur dudit dispositif.

2. Dispositif selon la revendication 1, dans lequel la boucle (3, 4) comporte au moins un aimant (7,11) pour le verrouillage de la boucle.

3. Dispositif selon l'une des revendications 1 ou 2, comportant un contact à piston pour l'activation du dispositif, une fois la ceinture fermée.

4. Dispositif selon l'une des revendications précédentes, comportant une source d'énergie rechargeable à travers la boucle (3), notamment par cordon USB (9).

5. Dispositif selon l'une des revendications précédentes, comportant un module inertiel six axes permettant de détecter les mouvements d'un porteur (6), le module inertiel comportant notamment un accéléromètre couplé à un gyroscope.

6. Dispositif selon l'une des revendications précédentes, dans laquelle la boucle (3) comporte une ouverture (21) agencé pour laisser passer un fil électrique (12) disposé pour transmettre une impulsion électrique donnée par le microcontrôleur pour autoriser un déclenchement de l'airbag en cas de chute.

7. Dispositif selon l'une des revendications précédentes, dans laquelle la boucle est formée de deux parties assemblables, une première partie (3) en forme de boîtier rectangulaire contenant l'électronique et une deuxième partie (4), ces deux parties comportant ensemble un corps cylindrique de connexion (15) comportant un dispositif de fixation à baïonnette (16).

8. Dispositif selon la revendication 6, dans lequel les côtés externes desdites parties (3, 4) comportent des ouvertures (19, 20) pour s'attacher aux extrémités de la poche (1) formant ceinture.

9. Dispositif selon l'une des revendications précédentes, comportant des moyens visuels (21) et/ou sonore (22) agencés pour indiquer l'état de fonctionnement dudit dispositif.

10. Dispositif selon l'une des revendications précédentes, comportant une boucle comprenant :
- une première partie de forme cylindrique comportant une rainure hélicoïdale s'étendant d'une extrémité de la première partie de boucle, la rainure étant courbée avec une encoche à sa seconde extrémité, et
- une seconde partie comportant une protubérance agencée pour coulisser dans ladite rainure et venir s'engager dans ladite encoche,
chaque partie de la boucle comportant un aimant agencée pour solidariser la première et la seconde partie de boucle, les deux dites parties de boucle étant désolidarisables l'une de l'autre par le déplacement angulaire d'une première partie de boucle par rapport à l'autre partie de boucle puis par le coulissement de la protubérance dans la rainure jusqu'à son extraction.

11. Dispositif selon l'une des revendications précédentes, où le microcontrôleur stocke un algorithme qui distingue entre les mouvements brusques qui correspondent à des chutes et d'autres mouvements qui ne correspondent pas à des chutes, pour définir le moment pour déclencher la source de gaz seulement en cas de chute.

12. Dispositif selon la revendication 1, dans lequel ledit module de communication sans-fil est apte à transmettre une information relative à la position du porteur et au déclenchement de l'airbag à une autre personne que le porteur dudit dispositif.

13. Dispositif selon la revendication 12, le dispositif comportant de surcroit un système de supervision, agencé pour permettre à une tierce personne de contrôler, d'une part un état du dispositif et d'autre part la géolocalisation du porteur, via le module de communication sans-fil.

## Patentansprüche

1. Sicherheitsvorrichtung zum Verhindern von Verletzungen an den Hüften, am Becken, am Gesäß und am Steißbein durch Aufblasen eines an einen Träger (6) angepassten Airbags, wobei die Vorrichtung enthält:
- eine tragbare aufblasbare Tasche (1);
- mindestens eine Quelle (2) freisetzbaren Gases, die mit der Tasche (1) gekoppelt ist;
- eine Gürtelschnalle (3, 4), in der eine Vielzahl von Sensoren angeordnet sind, die es ermöglichen, die Drehbewegung und die Beschleunigung eines Trägers (6) bei einem Sturz des Trägers zu bestimmen;
- einen Mikrocontroller, der geeignet ist, die von den verschiedenen Funktionsblöcken empfangenen Daten zu analysieren, um den Moment zum Auslösen der Gasquelle zu definieren; und
- eine Einrichtung, um die Quelle (2) zu entladen und das Gas in die Tasche (1) freizusetzen gemäß einer Bestimmung des Mikrocontrollers, dass der Träger fällt; **dadurch gekennzeichnet, dass**:
zwei der in der Schnalle angeordnete Sensoren ein erster Wärmesensor, der die Umgebungstemperatur misst, und ein zweiter Wärmesensor (5) sind, der die Körpertemperatur eines Trägers (6) misst, wobei die ersten und zweiten Wärmesensoren eingerichtet sind, um ein Auslösen des Airbags im Fall eines Sturzes nur zu erlauben, wenn die Vorrichtung von einem Träger getragen wird, durch die Messung der von den zwei Wärmesensoren gemessenen Temperaturdifferenz, um sich so der Anwesenheit eines Trägers der Vorrichtung zu vergewissern, und dass sie ein drahtloses Kommunikationsmodul aufweist, das fähig ist,
eine Information bezüglich eines Zustands der Vorrichtung entsprechend der Anwesenheit oder nicht eines Trägers und des Auslösens oder nicht des Airbags an eine andere Person als den Träger der Vorrichtung zu übertragen.

2. Vorrichtung nach Anspruch 1, wobei die Schnalle (3, 4) mindestens einen Magneten (7, 11) zur Verriegelung der Schnalle aufweist.

3. Vorrichtung nach einem der Ansprüche 1 oder 2, die einen Kolbenkontakt für die Aktivierung der Vorrichtung aufweist, wenn der Gürtel geschlossen ist.

4. Vorrichtung nach einem der vorhergehenden Ansprüche, die eine über die Schnalle (3) aufladbare Energiequelle aufweist, insbesondere durch USB-Kabel (9).

5. Vorrichtung nach einem der vorhergehenden Ansprüche, die ein sechsachsiges Trägheitsmodul aufweist, das es ermöglicht, die Bewegungen eines Trägers (6) zu erkennen, wobei das Trägheitsmodul insbesondere einen mit einem Gyroskop gekoppelten Beschleunigungsmesser aufweist.

6. Vorrichtung nach einem der vorhergehenden Ansprüche, wobei die Schnalle (3) eine Öffnung (21) aufweist, die eingerichtet ist, um ein Stromkabel (12) durchzulassen, das eingerichtet ist, um einen gegebenen Stromimpuls vom Mikrocontroller zu übertragen, um ein Auslösen des Airbags im Fall eines Sturzes zu erlauben.

7. Vorrichtung nach einem der vorhergehenden Ansprüche, wobei die Schnalle von zwei zusammensetzbaren Teilen gebildet wird, einem ersten Teil (3) in Form eines rechteckigen Gehäuses, das die Elektronik enthält, und einem zweiten Teil (4), wobei diese zwei Teile zusammen einen zylindrischen Verbindungskörper (15) aufweisen, der eine Bajonett-Befestigungsvorrichtung (16) aufweist.

8. Vorrichtung nach Anspruch 6, wobei die Außenseiten der Teile (3, 4) Öffnungen (19, 20) aufweisen, um sich an den Enden der Tasche (1) zu befestigen, die einen Gürtel bildet.

9. Vorrichtung nach einem der vorhergehenden Ansprüche, die optische (21) und/oder akustische Einrichtungen (22) aufweist, die eingerichtet sind, um den Betriebszustand der Vorrichtung anzuzeigen.

10. Vorrichtung nach einem der vorhergehenden Ansprüche, die eine Schnalle aufweist, die enthält:
- einen ersten Teil von zylindrischer Form, der eine Spiralnut aufweist, die sich von einem Ende des ersten Teils der Schnalle erstreckt, wobei die Nut gekrümmt ist, mit einer Einkerbung an ihren zweiten Ende, und
- einen zweiten Teil, der einen Vorsprung aufweist, der eingerichtet ist, um in der Nut zu gleiten und sich in die Einkerbung einzufügen,
wobei jeder Teil der Schnalle einen Magneten aufweist, der eingerichtet ist, um den ersten und den zweiten Schnallenteil fest miteinander zu verbinden, wobei die zwei Schnallenteile durch die Winkelverschiebung eines ersten Schnallenteils bezüglich des anderen Schnallenteils und dann dem Gleiten des Vorsprungs in der Nut bis zu seiner Entnahme voneinander gelöst werden können.

11. Vorrichtung nach einem der vorhergehenden Ansprüche, wobei der Mikrocontroller einen Algorithmus speichert, der zwischen den abrupten Bewegungen, die Stürzen entsprechen, und anderen Bewegungen unterscheidet, die nicht Stürzen entsprechen, um den Moment zum Auslösen der Gasquelle nur im Fall eines Sturzes zu definieren.

12. Vorrichtung nach Anspruch 1, wobei das drahtlose Kommunikationsmodul fähig ist, eine Information bezüglich der Position des Trägers und des Auslösens des Airbags an eine andere Person als den Träger der Vorrichtung zu übertragen.

13. Vorrichtung nach Anspruch 12, wobei die Vorrichtung zudem ein Überwachungssystem aufweist, das eingerichtet ist, um einer dritten Person zu erlauben, einerseits einen Zustand der Vorrichtung und andererseits die Geolokalisierung des Trägers über das drahtlose Kommunikationsmodul zu kontrollieren.

## Claims

1. A safety device for preventing injuries to the hips, the pelvis, the buttocks and the coccyx by inflation of an airbag adapted for wearer (6), said device comprising:
- a portable inflatable bag (1);
- at least one source (2) of releasable gas coupled to the bag (1);
- a belt buckle (3, 4) in which a plurality of sensors are arranged, for determining the angular movement and the acceleration of a wearer (6) during a fall of the wearer;
- a microcontroller adapted to analyse data received by different functional blocks in order to define the time to trigger the gas source; and
- means for discharging the source (2) to release the gas into the bag (1) according to a determination by the microcontroller that the wearer is falling;
**characterised in that**
two of the sensors arranged in the buckle are a first thermal sensor measuring the ambient temperature and a second thermal sensor (5) measuring the body temperature of a wearer (6), the first and second thermal sensors being arranged for triggering of the airbag in case of a fall only when the device is worn by a wearer, by measuring the temperature difference measured by the two thermal sensors thus ensuring the presence of a wearer of the device and **in that** it comprises a wireless communication module for transmitting information relative to a state of the device corresponding to the presence or not of a wearer and to the triggering of the airbag or not to a person other than the wearer of said device.

2. The device according to claim 1, wherein the buckle (3, 4) includes at least one magnet (7, 11) for locking the buckle.

3. The device according to any of claims 1 or 2, including a piston contact for activating the device, once the belt is closed.

4. The device according to any of the preceding claims, including a rechargeable energy source through the buckle (3), in particular by a USB cable (9).

5. The device according to any of the preceding claims, including a six-axis inertial module for detecting the movements of a wearer (6), the inertial module including in particular an accelerometer coupled to a gyroscope.

6. The device according to any of the preceding claims, wherein the buckle (3) includes an opening (21) arranged to pass an electric wire (12) disposed to transmit an electrical pulse given by the microcontroller for authorizing a triggering of the airbag in case of a fall.

7. The device according to any of the preceding claims, wherein the buckle is formed of two attachable portions, a first portion (3) in the form of a rectangular housing containing electronics and a second portion (4), these two portions together including a cylindrical connection body (15) including a bayonet fastening device (16).

8. The device according to claim 6, wherein the outer sides of said portions (3, 4) include openings (19, 20) to be attached to the ends of the bag (1) forming a belt.

9. The device according to any of the preceding claims, including visual (21) and/or sound (22) means arranged to indicate the operating state of said device.

10. The device according to any of the preceding claims, including a buckle comprising:
- a first cylindrically shaped portion including a helical groove extending from one end of the first buckle portion, the groove being curved with a notch at the second end thereof, and
- a second portion including a protrusion arranged to slide in said groove and to be engaged into said notch,
each portion of the buckle including a magnet arranged to hold together the first and second buckle portions, the two said buckle portions being seperable from each other by the angular displacement of a first buckle portion relative to the other buckle portion then by the sliding of the protrusion in the groove until the extraction thereof.

11. The device according to any of the preceding claims, wherein the microcontroller stores an algorithm which distinguishes between sudden movements which correspond to falls and other movements which do not correspond to falls, in order to define the time to trigger the gas source only in case of a fall.

12. The device according to claim 1, in which said wireless communication module is capable of transmitting information relative to the location of the wearer and to the triggering of the airbag to a person other than the wearer of said device.

13. The device according to claim 12, the device further comprising a supervision system configured to allow a third party to check, on one hand a state of the device and on the other hand the geolocation of the wearer, via the wireless communication module.
